# EUROPEAN PATENT SPECIFICATION

(11) **EP 3 764 794 B1**
(45) Date of publication and mention of the grant of the patent: **31.05.2023**
(21) Application number: 19711252.7
(22) Date of filing: 01.03.2019
(51) Int. Cl.: A01N 59/16, A01N 59/20, A01N 25/12, A01N 25/30, A01N 25/34, A01P 1/00

(54) **AN ANTIMICROBIAL PARTICULATE COMPOSITION AND A PERSONAL CARE COMPOSITION COMPRISING THE SAME**
ANTIMIKROBIELLE PARTIKELZUSAMMENSETZUNG UND DIE ENTHALTENDE KÖRPERPFLEGEZUSAMMENSETZUNG
COMPOSITION PARTICULAIRE ANTIMICROBIENNE ET COMPOSITION DE SOIN PERSONNEL LA COMPRENANT

(30) Priority: 12.03.2018 EP 18161216
(43) Date of publication of application: 20.01.2021
(73) Proprietor: Unilever Global IP Limited, Wirral, Merseyside CH62 4ZD (GB); Unilever IP Holdings B.V., 3013 AL Rotterdam (NL)
(72) Inventor: MITRA, Rupak, Bangalore 560 066 (IN); MURALIDHARAN, Girish, Bangalore 560 066 (IN); PRAMANIK, Amitava, Bangalore 560 066 (IN); SARKAR, Samarpita, Burdwan 713 304 West Bengal (IN); DAS, Somnath, Bangalore 560 066 (IN)
(74) Representative: van den Brom, Coenraad Richard
(86) International application number: PCT/EP2019/055102
(87) International publication number: WO 2019/174928

(56) References cited:
- WO-A1-2017/029482
- WO-A1-2018/114121
- JP-A- H0 717 803
- JP-B2- 2 998 061
- US-A- 5 169 682
- US-A1- 2017 209 347
- US-A1- 2018 305 228
- PRAKASH CHANDRA SAHOO ET AL: "Facile fabrication of silver nanoparticle embedded CaCO 3 microspheres via microalgae-templated CO 2 biomineralization: application in antimicrobial paint development", RSC ADVANCES, vol. 4, no. 61, 1 January 2014 (2014-01-01), page 32562, XP055478769, DOI: 10.1039/C4RA03623A

## Description

### Field of the invention

The present invention relates to an antimicrobial particulate composition and a process for preparing the same. The invention also relates to a personal care composition comprising the antimicrobial particulate composition. The invention is especially useful in that the antimicrobial particulate composition delivers the desired action even when the process to prepare it is scaled up to production scale while delivering enhanced leach of the antimicrobial metal species while ensuring that the antimicrobial particulate composition and the personal care composition thus prepared exhibit a desired light colour.

### Background of the invention

Any discussion of the prior art throughout the specification should in no way be considered as an admission that such prior art is widely known or forms part of the common general knowledge in the field.

Antimicrobial metal particles such as silver or copper nanoparticles are broad-spectrum antimicrobial materials used for different purposes including water purification and other hygiene-related products. However, these metal nanoparticles are known to be toxic and cannot be used as such in compositions for human use nor used at high concentrations. They also have an impact on the environment.

In a co-pending application, WO2016020168 (Unilever) discloses a process for preparing an antimicrobial particulate composition where metal nanoparticles are immobilized in an inorganic porous material and also for incorporating these particles in personal care or hygiene compositions. The antimicrobial particulate composition comprises antimicrobial metal particles immobilized on an inorganic porous material selected from zinc oxide, magnesium hydroxide or calcium carbonate. The object of the above invention was to form a light-coloured product while obtaining good antimicrobial property.

JP 2 998061 (Daiso Co LTD) relates to obtaining the subject substance exhibiting sufficient light resistance, antimicrobial effect and antimicrobial durability by adsorption-carrying antimicrobial metal fine particles obtained by a specific production method on the sun-face of porous calcium carbonate powder obtained by a specified production method. Particularly it relates to calcium carbonate powder obtained by adding a soluble phosphoric acid compound to an aqueous slurry of calcium carbonate and subsequently aging the mixture is brought into contact with the nonaqueous dispersion of antimicrobial metal fine particles to obtain the antimicrobial metal fine particlecarrying porous calcium carbonate having a BET ratio surface area of greater than or equal to1 00m<2> /g and an absorbed oil volume of greater than or equal to140cc/100g. The metal fine particles are preferably silver fine particles. The held amount of the metal fine particles is 0.001-10 weight percent based on the calcium carbonate. The calcium carbonate preferably has a cubic, fusiform or whisker-like shape. The solid content concentration of the calcium carbonate in the aqueous slurry is preferably 1-60 weight percent based on the weight of the calcium carbonate.

JP 70 17803 (Shiraishi Chuo Kenkyusho KK) relates to obtaining a powder of antimicrobial calcium carbonate which provides antimicrobial products with favorable looking and color by combining the powder with various media. Particularly, it relates to an antimicrobial calcium carbonate powder that has a porous structure with the peat like coagulation made of chain like micro particles, having a relative volume of 1.5ml/g or greater, a void volume of 1.0ml/g or greater, a BET relative surface area of 8m2/g or greater and stores metals such as silver, copper, zinc and the like or their water soluble compounds in the above mentioned porous structure. Since the antimicrobial calcium carbonate powder keeps an excellent antimicrobial activity for a long period, the powder can be applied to, for example, filtering materials, flooring materials, wall materials, coatings, toiletry products, food containers and the like to exhibit excellent antibacterial and antifungal activities.

Further, Prakash Chandra Sahoo et al (RSC Advances, 4, 61, page 32562) discloses vaterite-calcite microsphere having specific surface area of 39.1 m²/g embedded with silver nanoparticles (3.7 %) obtained via modified Tollens reaction.

US 5 169 682 (Asai Soichi) relates to a method of providing silver on a calcium carbonate material having fine pores, such as coral sand, oyster shells, and crab shells, including (a) contacting a calcium carbonate material with an aqueous solution of silver nitrate for a time effective for the silver nitrate to react with the calcium carbonate to form silver carbonate on the surface of the calcium carbonate material; (b) dewatering the calcium carbonate material after step (a); and (c) treating the calcium carbonate material after step (b) in an aqueous solution containing from 5 to 15 weight percent of a reducing agent selected from the group consisting of ascorbic acid, a derivative of ascorbic acid, erythrobic acid, and a derivative of erythrobic acid to reduce the silver carbonate on the surface of the calcium carbonate to silver. Preferably contacting is by injection of an upward stream of the aqueous solution of silver nitrate through a layer of the calcium carbonate material. Instead of or in addition to employing a reducing agent, the silver carbonate may be reduced directly by uniformly heating the calcium carbonate material after step (b) at a temperature of at least 200 DEG C., and preferably at a temperature ranging from 300 DEG to 400 DEG C.

WO 2017/029482 (Imerys Minerals LTD) relates to compositions comprising an inorganic particulate mineral and an antimicrobial metal, methods of making said compositions, use of said compositions in a coating composition or in a polymeric article, use of said compositions to inhibit the growth of one or more microbes and use of said compositions to eliminate one or more microbes, for example from a liquid.

One of the modes of working the invention disclosed in WO2016020168 was to use vaterite (a form of calcium carbonate) as the immobilizer. While the desired properties (light colour, good antimicrobial efficiency etc.) could be obtained when preparing the antimicrobial composition using the process at laboratory scale (of upto a few grams) using vaterite, the efficacy could not be duplicated when the process was scaled up to production scale conditions of about 100 kg of the material. The product so prepared at a production facility did not exhibit the desired leach of the metal species required for obtaining the expected antimicrobial activity. The reaction being kinetically driven, the present inventors envisage that due to the inherently long reaction times in a production scale process as compared to a laboratory scale process, the instability exhibited is much higher on scale up.

The present inventors through further experimentation at production scales determined that the kinetics of this reaction could be altered through use of certain polymers (as crystal habit modifiers) but found that the product so prepared suffers from the problem of low antimicrobial activity. The present inventors hypothesize that this low antimicrobial activity occurs since the antimicrobial metal species preferentially reacts with the polymer thus inhibiting the efficacy of embedding the metal species on the inorganic matrix.

The present inventors then attempted very many different strategies to solve this problem and finally arrived at a solution through use of a specific polymorph of calcium carbonate as the immobilizer. The use of this specific polymorph viz. calcite alone was not sufficient to solve all of the above mentioned problems. To obtain the desired leach of the metal species and thus the expected antimicrobial efficacy with the antimicrobial particulate composition prepared at production scales, the present inventors determined that only when the calcite has a specific surface area higher than a certain value, was it possible to get a product that solves all of the above problems. Surprisingly, not only were the above problems solved, but the product so obtained, had a light coloured appearance, as desired.

It is thus an object of the present invention to provide a light coloured antimicrobial particulate composition that has good antimicrobial properties, when prepared at production scale conditions.

### Summary of the invention

According to the first aspect of the present invention there is provided an antimicrobial particulate composition comprising:
(i) 0.05% to 3% by weight of antimicrobial metal particles immobilized on
(ii) 97 to 99.95% by weight of calcite having a specific surface area higher than 8 m²/g;
wherein the antimicrobial metal particles are silver or copper nanoparticles.

According to another aspect of the present invention there is provided a personal care composition comprising:
(i) 5% to 85% by weight of a surfactant and
(ii) 0.1 to 5% by weight of an antimicrobial particulate composition as per the first aspect of the present invention.

According to yet another aspect of the present invention there is provided a process for preparing an antimicrobial particulate composition, the process comprising the steps of:
(i) mixing 10- 50 wt% of an aqueous dispersion of calcite have a specific surface area higher than 8 m²/g and a particle size in the range of 1 to 10 microns in water, with an aqueous solution of 0.01- 10% of a reducing agent by weight of said calcite;
(ii) raising the temperature of the mixture of step (i) to a temperature in the range 60° C to 90°C;
(iii) adding 0.01 to 10% of a water soluble metal salt by weight of said calcite to the mixture of step (ii), wherein the water soluble metal salt is a water soluble salt of silver;
(iv) separating water from the product of step (iii) to prepare a wet cake; and
(v) drying the wet cake.

Yet another aspect of the present invention relates to a method of providing enhanced leaching of an antimicrobial metal on to a cleansing solution comprising dissolving/ dispersing the composition as per the first or the second aspect of the present invention in water.

Yet another aspect of the present invention relates to a non-therapeutic method of providing antimicrobial action on to a topical surface of a human or animal body comprising the steps of applying a composition of the first or second aspect on to the desired surface and optionally rinsing the surface substantially free of the composition with water.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The present invention relates to an antimicrobial particulate composition comprising an antimicrobial metal particles immobilized on calcite having a specific surface area higher than 8 m2/g.

The antimicrobial metal particles are silver or copper nanoparticles, preferably silver nanoparticles. As used herein, the term "metal particles" refers to particles of elemental metal, and excludes particles of metal salts,

metal oxides or other metal compounds. The antimicrobial metal particles are included in an amount of 0.05 to 3%, preferably 0.5 to 1.5% by weight of the antimicrobial particulate composition.

Calcite having a specific surface area higher than 8 m²/g is included in the antimicrobial particulate composition. The specific surface area is preferably higher than 12 m²/g, more preferably higher than 16 m²/g. The calcite polymorph is an especially important element of the antimicrobial particulate composition as it is seen that this particular polymorph is stable at the high temperatures during the process of embedding the antimicrobial metal particle in it. This stability is not observed when other polymorphs of calcium carbonate like vaterite or aragonite are used. Therefore the calcite used is pure calcite. Mixtures of calcite with other polymorphs like vaterite or argonite tend to be unsuitable for the present purpose. Ideally, pure calcite comprises 99.9% or even 100% calcite having a surface area higher than 8 m²/g. Further, the enhanced leaching is obtained only when high surface area calcite is used (specific surface area higher than 8 m²/g) while when calcium carbonate with low surface area like fine ground natural chalk (FGNC) or precipitated calcium carbonate (PCC) are used, efficacy is lower. Further, when calcium carbonate is reacted with certain phosphates, it is found to have substantial amounts of calcium phosphate in the matrix, and is therefore unsuitable for use in the present invention.

Calcite is included in 97 to 99.95%, preferably 98.5 to 99.5% by weight of the antimicrobial particulate composition. Calcite with a particle size in the range of 1 to 10 microns is preferably used, more preferably in the size range of 2 to 5 microns.

The specific surface area is measured as per this invention using the following method: Brunauer-Emmett-Teller (BET) Method was used to quantify the specific surface area of calcite. Nitrogen gas adsorption-desorption isotherms were recorded at 77 K. Specific surface area was calculated using pressure in the range of from 0.05 to 0.3 (P/Po) using the standard BET equation.

The particle size and calcite morphology was characterized using Field-Emission Scanning Electron Microscopy (FESEM). Ultra 55 (Zeiss) was used for FESEM imaging and operated at electron voltage of 2 kV. XRD was done to characterize the crystal structure of calcium carbonate.

In general, when reference is made to immobilized antimicrobial particles or immobilized antimicrobial metal particles or immobilized metal nanoparticles or immobilized materials with reference to specific metals such as silver or copper, it refers to antimicrobial particulate composition of the invention. The phrases 'antimicrobial metal particles' and 'metal species' are used interchangeably throughout this specification.

The invention also relates to a personal care composition comprising the antimicrobial particulate composition and a surfactant. The personal care composition includes 5% to 85%, preferably 15 to 40% by weight of a surfactant. The antimicrobial particulate composition of the present invention is included in amounts of 0.1 to 5%, preferably 0.25 to 4% by weight of the personal care composition.

The surfactant is selected from one or more from the class of anionic, non-ionic, cationic or zwitterionic surfactants, preferably from anionic surfactants. The anionic surfactant is preferably soap. The term soap means salt of fatty acid. Preferably, the soap is soap of C₈ to C₂₄ fatty acids, more preferably of C₁₀ to C₁₈ fatty acids. It is particularly preferred that the soap includes at least 40 wt% soap of C₈ to C₁₄ fatty acid, more preferably at least 50 wt% and most preferably at least 70 wt% of the total soap content. It is also preferred that when the personal care composition of the invention is formulated as a cleansing bar, it includes at most 60 wt% of the soap of C₁₆ to C₂₂ fatty acid, preferably at most 50 wt% and most preferably at most 30 wt% of the total soap content.

The cation may be an alkali metal, alkaline earth metal or ammonium ion, preferably alkali metals. Preferably, the cation is selected from sodium or potassium. The soap may be saturated or unsaturated. Saturated soaps are preferred over unsaturated soaps for stability. The oil or fatty acids may be of vegetable or animal origin.

The soap may be obtained by saponification of oils, fats or fatty acids. The fats or oils generally used to make soap bars may be selected from tallow, tallow stearins, palm oil, palm stearins, soya bean oil, fish oil, castor oil, rice bran oil, sunflower oil, coconut oil, babassu oil, and palm kernel oil. The fatty acids may be from coconut, rice bran, groundnut, tallow, palm, palm kernel, cotton seed or soyabean.

The fatty acid soaps may also be synthetically prepared (e.g. by the oxidation of petroleum or by the hydrogenation of carbon monoxide by the Fischer-Tropsch process). Resin acids, such as those present in tall oil, may also be used. Naphthenic acids may also be used. Soap is preferably included in 5 to 85%, preferably from 10 to 85%, more preferably 25 to 75% soap by weight of the personal care composition.

The personal care composition of the present invention can be in the form of a liquid, a gel, a cream or a solid composition. It is preferably in the form of a solid composition, more preferably in the form of a shaped solid for example a bar. The personal care composition of the invention may be a leave on or a wash off composition, preferably it is a wash off composition. By a leave on composition is meant that the composition is applied on the desired topical surface of the human body and left thereon for the desired action and not washed off till the person has a shower or a bath next. Leave on products therefore are left on the topical surface for several hours and possibly up to a whole day. Wash off products have sufficient amount of surfactants included therein that it is used for cleansing the desired topical surface e.g. the whole body, the hair and scalp or the face. It is applied on the topical surface and left thereon only for a few seconds or minutes and washed off thereafter with copious amounts of water. The personal care composition of the invention is preferably a wash off composition.

The inventors have determined that when the antimicrobial particulate composition of the invention is included in a surfactant (e.g soap) based composition, the antimicrobial action occurs in very short times e.g of the order of less than 5 minutes, preferably less than 2 minutes, further more preferably less than a minute and in many cases as low as in 10 seconds. Fortuitously wash off compositions include a surfactant for the cleaning action where surfaces are washed with a composition only for such short periods of time where not only the surface is washed clean of all undesirable dirt but one gets the benefit of the antimicrobial activity delivered by the composition of the invention, during this short period of time.

The personal care composition of the invention when presented as a transparent soap bar may include a water soluble organic solvent. This is preferably selected from the group consisting of polyol, hydrotropes or mixtures thereof. The water soluble organic solvent is preferably in the range of 20 to 45 wt%, more preferably in the range of 25% to 40 wt%, and most preferably in the range of 30 to 40 wt% based on the weight of the composition.

Preferred cleansing bar includes 20% to 45 wt% polyols based on the weight of the composition. Preferred polyols include one or more of glycerol, sorbitol, propylene glycol or polyethylene glycol. Usually a mixture is used. More preferred bar includes 25 to 40 wt% polyols and most preferred bars include 30 to 40 wt% of polyols. Polyhydric alcohols (polyols), such as propylene glycol, may serve as diluents to thin out the otherwise thick mixture of caustic soda and fatty acids.

Hydrotopes may also be included in the personal care composition of the invention. Hydrotopes include but are not limited to sodium cumene sulphonate, sodium toluene sulphonate, sodium xylene sulphonate & sodium alkyl aryl sulfonate, their derivatives and combinations thereof.

Electrolyte may optionally be included in the personal care composition of the invention. They are preferably included in the range of 3 to 20 wt%, more preferably in the range of 3.5 to 15 wt % and most preferably in range of 4 to 10% by weight of the composition. Preferred electrolytes of the present invention include sodium sulfate, sodium chloride, sodium acetate, sodium citrate, potassium chloride, potassium sulfate, sodium carbonate and other mono or di or tri salts of alkaline earth metals, more preferred electrolytes are sodium chloride, sodium sulfate, sodium citrate, potassium chloride and especially preferred electrolytes are sodium chloride, sodium sulfate, and sodium citrate and combinations thereof. For the avoidance of doubt, it is clarified that the electrolyte is a non-soap material.

An opacifier may be optionally present in the personal care composition. When opacifiers are present, the cleansing bar is generally opaque. Examples of opacifiers include titanium dioxide, zinc oxide and the like. A particularly preferred opacifier that can be employed when an opaque soap composition is desired is ethylene glycol mono- or distearate, for example in the form of a 20% solution in sodium lauryl ether sulphate. An alternative opacifying agent is zinc stearate.

The product can take the form of a water-clear, i.e. transparent soap, in which case it will not contain an opacifier, or alternatively, it can take the form of an opaque liquid soap containing an opacifier such as that herein defined.

Preferred personal care composition in the form of a cleansing bar includes 20 to 40 wt% water, more preferably 20 to 35 wt% and most preferably 22 to 30 wt% water based on the weight of the composition. More or less water may adversely affect transparency.

The pH of preferred soaps bars of the invention is from 8 to 11, more preferably 9 to 11.

A preferred personal care composition in the form of a bar may include up to 30 wt% benefit agents. Preferred benefit agents are moisturizers, emollients, sunscreens and anti-ageing compounds. The agents may be added at an appropriate step during the process of making the bars. Some benefit agents may be introduced as macro domains.

Other optional ingredients like anti-oxidants, perfumes, polymers, chelating agents, colourants, deodorants, dyes, emollients, moisturizers, enzymes, foam boosters, germicides, additional anti-microbials, lathering agents, pearlescers, skin conditioners, stabilisers, superfatting agents, sunscreens may be added in suitable amounts in the process of the invention. Preferably, the ingredients are added after the saponification step and before filtering. Sodium metabisulphite, ethylene diamine tetra acetic acid (EDTA), borax or ethylene hydroxy diphosphonic acid (EHDP) are preferably added to the formulation.

The process of the present invention for preparing an antimicrobial particulate composition, comprises the steps of:
i. mixing 10- 50 wt%, preferably 20 to 40wt% of an aqueous dispersion of calcite have a specific surface area higher than 8 m²/g and a particle size in the range of 1 to 10 microns in water, with an aqueous solution of 0.01- 10%, preferably 0.1 to 5% of a reducing agent by weight of said calcite;
ii. raising the temperature of the mixture of step (i) to a temperature in the range 60°C to 90°C, preferably 65 to 85°C;
iii. adding 0.01 to 10%, preferably 0.1 to 5% of a water soluble metal salt by weight of said calcite to the mixture of step (ii);
iv. separating the product of step (iii) from water to prepare a wet cake; and
v. drying the wet cake.

A calcination step is preferably not required for the process according to the invention and preferably the process does not comprise a calcination step.

The reducing agent for use in the process of the invention is preferably selected from water soluble salt of a carboxylic acid with a 1-4 carboxylate group. It is more preferably selected from sodium acetate, sodium oxalate, trisodium citrate or disodium ethylene diamine tetra acetate. It is preferred to include trisodium citrate as the reducing agent. The reducing agent is preferably added in the process as an aqueous solution, preferably as a 0.1 to 10%, more preferably as a 0.5 to 5% by weight of the solution.

It is highly preferred that the aqueous dispersion of the calcite and the aqueous solution of the reducing agent are mixed before adding the metal salt solution. The pH of the reaction medium during the process is maintained preferably at pH greater than 5 and more preferably is in the range 6 - 8.

The water soluble metal salt is selected from water soluble salt of silver. It is preferable to add the water soluble metal salt as an aqueous solution.

It is preferable that the water soluble silver salt is selected from silver nitrate or silver acetate. The concentration of the aqueous solution of water soluble metal salt is preferably in the range of 0.1 to 10%, more preferably 0.5 to 5% by weight of the aqueous solution. It is preferred to use silver nitrate as the water soluble salt of silver.

The invention also relates to a method of providing enhanced leaching of an antimicrobial metal on to a cleansing solution which comprises the step of dissolving/ dispersing the composition of the first aspect or the second aspect in water. The rate of leaching of antimicrobial metal species as per the present invention is higher than that obtained when the metal is immobilized on any other polymorph of calcium carbonate or on any other inorganic substrate e.g zinc oxide, magnesium oxide, titanium dioxide etc.

Yet another aspect of the invention provides for a non-therapeutic method of providing antimicrobial action on to a topical surface of a human or animal body comprising the steps of applying a composition of the first aspect or the second aspect on to the desired surface and optionally rinsing the surface substantially free of the composition, with water. By "substantially free of the composition" is meant that the composition is not perceptible to touch by a human hand. The composition of the first aspect of the invention is a particulate composition and is generally perceptible as a powdery, rough or granular tactile sensation when touched with the human hand. The composition of the second aspect of the invention is a personal care composition which comprises a surfactant, and is generally perceptible as a soapy or slippery feeling when touched by the human hand. When the surface is substantially free of the composition, one does not perceive such tactile sensations with the human hand. The composition is preferably applied on to the surface after dilution with water. The step of rinsing is optional but preferred. The step of rinsing is preferably carried out within 5 minutes of application of the composition, preferably within two minutes, further more preferably within a minutes, even further preferably within 30 seconds and in certain cases could be done within 10 or 15 seconds.

The invention will now be illustrated by means of the following non-limiting examples.

### Examples

### Example A, B, 1: Effect of the type of calcium carbonate and specific surface area on the leach of silver

### Preparation of antimicrobial particulate composition comprising silver nanoparticles

### Preparation of the calcium carbonate:

A. Calcite, comparative.
   FGNC (Fine Ground Natural Chalk) was procured from OmyaCarb. The particle size is 6 micron and the specific surface area is 2.1 m²/g
B. Vaterite (prepared in laboratory scale), comparative.
   2.4 g of calcium nitrate was taken in 2.5 ml water. 1.0 g of sodium carbonate was dissolved in 2.5 ml in water. In a glass beaker 2.5 ml of water was taken and the calcium nitrate solution was added. To this mixture, the sodium carbonate solution was added under constant stirring with an ice bath (~ 10 deg C) and filtered immediately under suction. The ratio of calcium: carbonate was maintained at 3:2.

### 1. Forcal U

Forcal U is a high surface area calcite which was purchased from Saurashtra Solid Private Ltd, India. The particle size ranged from 5±1 micron with the specific surface area of 20±2 m²/g.

### Preparation of the antimicrobial particulate composition comprising silver nanoparticles:

10 kg of calcium carbonate was dispersed in 10 liter water and mixed with 3.1 liter of 1% trisodium citrate solution. The mixture was heated to 70°C followed by the addition of freshly-prepared 2.1 liter of 1% silver nitrate solution. The mixture was stirred for 20 minutes at 300 rpm. The sample was filtered and dried at room temperature. The size of the silver nanoparticles formed in the above process ranged between 5-50 nm and these particles were immobilized in the respective calcium carbonate.

### Evidence for silver incorporation in the calcium carbonate:

Energy-dispersive X-ray spectroscopic analysis of immobilized silver particle in zinc oxide prepared by the process described in Example 1 was carried out using a FESEM instrument (ZEISS) operated at 10 kV electron voltage to determine the presence of silver in the immobilized material. The analyzed weight% of different ingredients is shown in table 1 below:

**Table 1**

| **Element** | **Weight (%)** |
|---|---|
| Carbon | 31.83 |
| Oxygen | 27.65 |
| Calcium | 35.64 |
| Silver | 4.88 |
| Total | 100.00 |

Results in table 1 show that the material contains carbon, calcium, oxygen and silver as constituents in the immobilized silver particles.

### Silver leaching kinetics

The procedure to measure the leach kinetics of the various samples prepared above is give below:
One litre of dispersion containing silver on the calcium carbonate equivalent to 1 ppm of Ag was prepared and small aliquots (~10 ml) were collected at 3 different time points, namely, 10 s, 30s and 60 s. The samples were immediately filtered through Whatman filter paper 41 and digested with concentrated Nitric acid for ICP analysis. The silver content was calculated by using the standard calibration plot.

The data on the leach kinetics is given in the Table 2 below.

**Table 2**

| Material | Time (s) | | |
|---|---|---|---|
| | 10 | 30 | 60 |
| Example A | 164 ppb | 248 ppb | 226 ppb |
| Example B | 158 ppb | 351 ppb | 499 ppb |
| Example 1 | 226 ppb | 459 ppb | 709 ppb |

The data in table 2 above indicates that antimicrobial particulate composition as per the invention (Example -1) delivers vastly superior leach of silver as compared to the calcium carbonate used in the past (Comparative example - B). Also the data indicates that the specific surface area of the calcite is critical to obtaining the high leach rate (Example 1 as compared to Comparative Example A).

### Examples C, D, 2, 3: Antimicrobial efficacy:

The antimicrobial efficacy was tested using gram positive bacteria *Staphylococcus aureus* by using Time kill assay according to BS EN1040 protocol.

10 g soap was dissolved in 90 mL water at 50°C. It was allowed to equilibrate to a temperature of 40°C in a water bath. Antimicrobial particulate composition comprising silver nanoparticles was added to the soap solution to get 1 or 2 ppm effective silver load (as listed below) and the same was calculated by ICP-OES analysis. 1 mL bacterial suspension of *Staphylococcus aureus* (10⁸ cells/mL) was added to the tubes containing 1 mL sterile water and was allowed to equilibrate at 40°C for 2 minutes. 8 mL of 10% soap solution containing antimicrobial particulate composition comprising silver nanoparticles was added to these bacterial suspensions respectively and incubated for 10 seconds and 30 seconds respectively. On these respective time points, 1mL suspension was taken and added to 9 mL of neutralizer (Dey-Engley Neutralizing Broth) to inactivate the action of the antimicrobial. The neutralized samples were then plated after serial dilution in Trypticase Soy Agar (nutrient medium) to enumerate the residual bacteria. The various samples prepared are given below and the log reduction obtained with them are presented in Table 3.

Comparative Example C: White milled soap bar.

Comparative Example D: Soap with silver embedded in vaterite (as given below): Silver content - 1 ppm.

### Vaterite (prepared at large scale)

100 g of vaterite was prepared by mixing 500 ml each of 2M Ca(NO₃)₂ and 2M Na₂CO₃ in the presence of 240 ml of 1% glycine or PSS . The slurry was mixed for 10 mins, filtered, washed with deionised water and dried in air. The particle size is 6±2 micron and the specific surface area is measured to be 4.1 m²/g.
Example 2: Soap with silver embedded in high surface area calcite (as per Example 1): Silver content - 1 ppm
Example 3: Soap with silver embedded in high surface area calcite (as per Example 1): Silver content - 2 ppm

The various samples above were tested for log reduction of *S*. *Aureus* as per the procedure described earlier. The log reduction at 10 seconds and 30 seconds contact time for the above samples is summarized in Table 3 below:

**Table 3**

| Material | Log reduction | |
|---|---|---|
| | 10 s | 30 s |
| Example C | - | 0.008 |
| Example D | 0.14 | 0.04 |
| Example 2 | 0.61 | 2.35 |
| Example 3 | 1.16 | 2.64 |

The data in table 3 shows that antimicrobial particulate composition as per the invention in combination with soap (Example 2 and 3) have superior bacterial reduction against *S.aureus* as compared to compositions outside the invention.

### Examples E, 4: Antibacterial efficacy of soap composition as per the invention as compared to a conventional soap bar containing chelated silver.

Comparative Example E: A conventional soap bar was prepared which contained silver chelated with diethylene triamine pentaacetic acid (DTPA).

Example 4: A soap bar was prepared as per the invention containing silver embedded in calcite.

Bars of soap having following the composition as shown in Table 4 below were made by the well-known milling and plodding process. To this antimicrobial particulate composition of Comparative Example E and 4, respectively, were added.

**Table 4**

| Ingredient | Wt% |
|---|---|
| Sodium palmate and Sodium palm kernelate (85:15 parts w/w) | 68.0 |
| Alpha olefin sulfonate | 1.0 |
| Talc | 6.0 |
| Glycerine | 6.0 |
| Water and other minors | To 100 |

The amount of silver in the soap bars was analysed via Inductively Coupled Plasma (ICP) analysis. 1 g of soap bar was grated and transferred to a Teflon^{®} flask. The sample was digested using 4 ml of Suprapur^{®} nitric acid, 2 ml of 30% Hydrogen peroxide and 4 ml water. The sample was microwave digested at 150 °C for 15 minutes. The tube was cooled gradually to room temperature. The sample was made up to 50 ml followed by filtration through 0.22 µm nylon syringe filter. Standard calibration samples from 100 ppb to 5 ppm of Ag were prepared and the silver content in the sample was measured from the calibration plot.

The amount of silver in soap bars with Example E [a sample size of three bars] was found to be 4.76 ± 0.07 ppm. The amount of silver in soap bars with Example 4 [across a sample size of three bars] was found to be 1.9 ± 0.1 ppm.

The soap samples were tested for antimicrobial activity against *S*. *Aureus* as per the procedure given below:
The test is a standard method for assessment of antimicrobial activity for water-miscible compounds using a time-kill procedure. The concerned reference number is ASTM E2783-11 and details thereof are as follows.

An 8 % w/v solution of the concerned soap was prepared by dissolving 8 g of the soap in 92 ml sterile distilled water (pre-warmed to 50 ± 2 °C). The soap solution was equilibrated to a temperature of 40°C in a water bath. 1 mL bacterial suspension of *Staphylococcus aureus* (10⁸ cells/mL), was added to the tubes containing 1 mL sterile water and was allowed to equilibrate at 40°C for 2 minutes. 8 mL of 10% soap solution containing antimicrobial particulate composition comprising silver nanoparticles was added to these bacterial suspensions respectively and incubated for 10 seconds and 30 seconds respectively. On these respective time points, 1mL suspension was taken and added to 9 mL of neutralizer (Dey-Engley Neutralizing Broth) to inactivate the action of the antimicrobial. The samples were then further diluted in steps of 10-fold dilution by repeating the above step. Thereafter, an aliquot of 1 ml was plated with TSA in duplicate which resulted in final dilution of 10⁻¹, 10⁻², 10⁻³, 10⁻⁴ and 10⁻⁵. The plates were incubated at 35 ± 2 °C for 24 hours or until sufficient growth of *S*. *aureus* was observed. It was expected that each bar of soap would bring about some reduction in the viable bacterial count. Such reduction is usually expressed in terms of log₁₀ reduction.

A 4-Log Reduction on a surface with 1,000,000 CFUs would leave 100 CFUs, which is written or expressed as a 99.99% reduction in potentially harmful microorganisms. A reduction of 1 log (90%) reduces CFUs on a test area from 1,000,000 CFUs to 100,000 and 2 log (99%) reduces 1,000,000 to 10,000.

The log reduction obtained using the above procedure from the two soap samples are given in the table 5 below:

**Table 5**

| Material | Log reduction | |
|---|---|---|
| | 10 s | 30 s |
| Example E (4.8 ppm silver) | 0.8 | 2.5 |
| Example 4 (1.9 ppm Silver) | 0.8 | 2.3 |

The data in Table -5 above indicates that the composition as per the present invention (Example 4) is capable of providing similar antimicrobial activity as compared to a conventional soap containing much higher silver content (Comparative example E).

## Claims

1. An antimicrobial particulate composition comprising:
i. 0.05% to 3% by weight of antimicrobial metal particles immobilized on
ii. 97 to 99.95% by weight of calcite having a specific surface area higher than 8 m²/g;
wherein the antimicrobial metal particles are silver or copper nanoparticles.

2. An antimicrobial particulate composition as claimed in claim 1 wherein the antimicrobial metal particles are silver nanoparticles.

3. A personal care composition comprising:
(i) 5% to 85% by weight of a surfactant and
(ii) 0.1 to 5% by weight of an antimicrobial particulate composition as claimed in claims 1 or 2.

4. A personal care composition as claimed in claim 3 wherein the surfactant is an anionic surfactant preferably soap.

5. A process for preparing an antimicrobial particulate composition, the process comprising the steps of:
i. mixing 10- 50 wt% of an aqueous dispersion of calcite have a specific surface area higher than 8 m²/g and a particle size in the range of 1 to 10 microns in water, with an aqueous solution of 0.01- 10% of a reducing agent by weight of said calcite;
ii. raising the temperature of the mixture of step (i) to a temperature in the range 60° C to 90°C;
iii. adding 0.01 to 10% of a water soluble metal salt by weight of said calcite to the mixture of step (ii), wherein the water soluble metal salt is a water soluble salt of silver;
iv. separating water from the product of step (iii) to prepare a wet cake; and
v. drying the wet cake.

6. A process as claimed in claim 5 wherein the water soluble salt of silver is selected from silver nitrate or silver acetate.

7. A process as claimed in any one of claims 5 or 6 wherein the reducing agent is selected from a water soluble salt of a carboxylic acid with a 1-4 carboxylate group.

8. A process as claimed in claim 7 wherein the reducing agent is selected from sodium acetate, sodium oxalate, trisodium citrate or disodium ethylene diamine tetra acetate.

9. A method of providing enhanced leaching of an antimicrobial metal on to a cleansing solution comprising dissolving/ dispersing a composition as claimed in any one of claims 1 to 4 in water.

10. A non-therapeutic method of providing antimicrobial action on to a topical surface of a human or animal body comprising the steps of applying a composition as claimed in any one of claims 1 to 4 on to the desired surface and optionally rinsing the surface substantially free of the composition with water.

11. A method as claimed in claim 10 wherein the step of rinsing is carried out 10 seconds to 5 minutes after the composition is applied on to the desired surface.

12. An antimicrobial particulate composition as claimed in claims 1 or 2 obtainable by a process as claimed in any one of claims 5 to 9.

## Patentansprüche

1. Antimikrobielle Partikelzusammensetzung, umfassend:
i. 0,05 bis 3 Gewichts-% antimikrobielle Metallpartikel, immobilisiert auf
ii. 97 bis 99,95 Gewichts-% Calcit einer spezifischen Oberfläche von mehr als 8 m²/g,
wobei die antimikrobiellen Metallpartikel Silber- oder Kupfer-Nanopartikel sind.

2. Antimikrobielle Partikelzusammensetzung, wie im Anspruch 1 beansprucht, wobei die antimikrobiellen Metallpartikel Silber-Nanopartikel sind.

3. Körperpflegezusammensetzung, umfassend:
(i) 5 bis 85 Gewichts-% eines Tensids und
(ii) 0,1 bis 5 Gewichts-% einer antimikrobiellen Partikelzusammensetzung, wie in den Ansprüchen 1 oder 2 beansprucht.

4. Körperpflegezusammensetzung, wie im Anspruch 3 beansprucht, wobei das Tensid ein anionisches Tensid, vorzugsweise Seife ist.

5. Verfahren zur Herstellung einer antimikrobiellen Partikelzusammensetzung, wobei das Verfahren die Schritte umfasst:
i. Mischen von 10 - 50 Gew.-% einer wässrigen Dispersion von Calcit mit einer spezifischen Oberfläche von mehr als 8 m²/g und einer Partikelgröße in dem Bereich von 1 bis 10 Mikrometer in Wasser mit einer wässrigen Lösung von 0,01 - 10% eines Reduktionsmittels, bezogen auf das Gewicht des Calcits;
ii. Erhöhen der Temperatur der Mischung des Schritts (i) auf eine Temperatur in dem Bereich von 60°C bis 90°C;
iii. Hinzufügen von 0,01 bis 10% eines linearen wasserlöslichen Metallsalzes, bezogen auf das Gewicht des Calcits, zu der Mischung von Schritt (ii), wobei das wasserlösliche Metallsalz ein wasserlösliches Silbersalz ist;
iv. Abtrennen von Wasser aus dem Produkt vom Schritt (iii), um einen nassen Kuchen herzustellen, und
v. Trocknen des nassen Kuchens.

6. Verfahren, wie im Anspruch 5 beansprucht, wobei das wasserlösliche Silbersalz aus Silbernitrat oder Silberacetat ausgewählt ist.

7. Verfahren, wie in irgendeinem der Ansprüche 5 oder 6 beansprucht, wobei das Reduktionsmittel aus einem wasserlöslichen Salz einer Carbonsäure mit 1 bis 4 Carboxylatgruppen ausgewählt ist.

8. Verfahren, wie im Anspruch 7 beansprucht, wobei das Reduktionsmittel aus Natriumacetat, Natriumoxalat, Trinatriumcitrat oder Dinatriumethylendiamintetraacetat ausgewählt ist.

9. Verfahren zum Bereitstellen einer verbesserten Auslaugung eines antimikrobiellen Metalls auf einer Reinigungslösung, umfassend das Auflösen/Dispergieren einer Zusammensetzung, wie in irgendeinem der Ansprüche 1 bis 4 beansprucht, in Wasser.

10. Nicht-therapeutisches Verfahren zum Bereitstellen einer antimikrobiellen Wirkung auf einer topischen Oberfläche eines menschlichen oder tierischen Körpers, umfassend die Schritte des Auftragens einer Zusammensetzung, wie in irgendeinem der Ansprüche 1 bis 4 beansprucht, auf die gewünschte Oberfläche und gegebenenfalls im Wesentlichen Freispülen der Oberfläche von der Zusammensetzung mit Wasser.

11. Verfahren nach Anspruch 10, wobei der Schritt des Spülens 10 Sekunden bis 5 Minuten nach dem Auftragen der Zusammensetzung auf die gewünschte Oberfläche durchgeführt wird.

12. Antimikrobielle Partikelzusammensetzung, wie in den Ansprüchen 1 oder 2 beansprucht, erhältlich durch ein Verfahren, wie in irgendeinem der Ansprüche 5 bis 9 beansprucht.

## Revendications

1. Composition particulaire antimicrobienne comprenant :
i. 0,05 % à 3 % en masse de particules de métal antimicrobiennes immobilisées sur
ii. 97 à 99,95 % en masse de calcite ayant une surface spécifique supérieure à 8 m²/g ;
dans laquelle les particules de métal antimicrobiennes sont des nanoparticules d'argent ou de cuivre.

2. Composition particulaire antimicrobienne selon la revendication 1, dans laquelle les particules de métal antimicrobiennes sont des nanoparticules d'argent.

3. Composition pour le soin de la personne comprenant :
(i) 5 % à 85 % en masse d'un tensioactif et
(ii) 0,1 à 5 % en masse d'une composition particulaire antimicrobienne selon la revendication 1 ou 2.

4. Composition pour le soin de la personne selon la revendication 3, dans laquelle le tensioactif est un tensioactif anionique de préférence du savon.

5. Procédé pour la préparation d'une composition particulaire antimicrobienne, le procédé comprenant les étapes de :
i. mélange de 10-50 % en masse d'une dispersion aqueuse de calcite ayant une surface spécifique supérieure à 8 m²/g et une taille de particule dans l'intervalle de 1 à 10 microns dans l'eau, avec une solution aqueuse de 0,01-10 % d'un agent réducteur en masse de ladite calcite ;
ii. élévation de la température du mélange de l'étape (i) à une température dans l'intervalle de 60°C à 90°C ;
iii. addition de 0,01 à 10 % d'un sel de métal soluble dans l'eau en masse de ladite calcite au mélange de l'étape (ii), dans lequel le sel de métal soluble dans l'eau est un sel d'argent soluble dans l'eau ;
iv. séparation de l'eau du produit de l'étape (iii) pour préparer un gâteau humide ; et
v. séchage du gâteau humide.

6. Procédé selon la revendication 5, dans lequel le sel d'argent soluble dans l'eau est choisi parmi le nitrate d'argent ou l'acétate d'argent.

7. Procédé selon l'une quelconque des revendications 5 ou 6, dans lequel l'agent réducteur est choisi parmi un sel soluble dans l'eau d'un acide carboxylique avec un groupe 1-4 carboxylate.

8. Procédé selon la revendication 7, dans lequel l'agent réducteur est choisi parmi l'acétate de sodium, oxalate de sodium, citrate trisodique ou tétraacétate d'éthylène diamine disodique.

9. Procédé de fourniture de lessivage amélioré d'un métal antimicrobien sur une solution de nettoyage comprenant la dissolution/ dispersion d'une composition selon l'une quelconque des revendications 1 à 4 dans de l'eau.

10. Procédé non-thérapeutique de fourniture d'une action antimicrobienne sur une surface topique d'un corps humain ou animal comprenant les étapes d'application d'une composition selon l'une quelconque des revendications 1 à 4 sur la surface souhaitée et de rinçage éventuel de la surface substantiellement exempte de la composition avec de l'eau.

11. Procédé selon la revendication 10, dans lequel l'étape de rinçage est réalisée pendant de 10 secondes à 5 minutes après que la composition est appliquée sur la surface souhaitée.

12. Composition particulaire antimicrobienne selon la revendication 1 ou 2 pouvant être obtenue par un procédé selon l'une quelconque des revendications 5 à 9.
